Europäisches Patentamt

(19) European Patent Office

Office européen des brevets

(11) EP 0 775 493 A2

## (12) EUROPEAN PATENT APPLICATION

(43) Date of publication:
28.05.1997 Bulletin 1997/22

(51) Int. Cl.⁶: **A61K 35/14**, A61M 1/34

(21) Application number: 96118698.8

(22) Date of filing: 21.11.1996

(84) Designated Contracting States:
AT BE CH DE DK ES FR GB IT LI NL SE

(30) Priority: 22.11.1995 US 561780

(71) Applicant: BAXTER INTERNATIONAL INC.
Deerfield, IL 60015-4633 (US)

(72) Inventor: Muller-Derlich, Jutta
82110 Germering (DE)

(74) Representative: MacGregor, Gordon et al
ERIC POTTER CLARKSON
St. Mary's Court
St. Mary's Gate
Nottingham, NG1 1LE (GB)

(54) **Extracorporeal xenogeneic organ perfusion following antibody depletion by immunoapheresis**

(57) The invention provides a method for treating a patient in need of ex vivo perfusion of his/her blood through a pig organ comprising first passing plasma from the patient over a column coupled to anti-human immunoglobulin antibodies, thereby removing a significant portion of the immunoglobulin from the patient's plasma, recombining the patient's immunoglobulin-depleted plasma with the patient's red blood cells, perfusing the patient's immunoglobulin-depleted blood through the pig organ, and then reinfusing the blood to the patient. Depletion of immunoglobulin removes anti-pig natural antibodies which would otherwise cause a hyperacute rejection reaction in the pig organ, thus quickly destroying the organ within 2-6 hours. The invention allows a single pig organ to function well in ex vivo perfusion for a much longer time, without signs of hyperacute rejection in the organ. The invention is especially useful in cases of acute liver failure where ex vivo perfusion of the patient's blood through a pig liver can bring the patient out of hepatic coma and bridge the patient to liver transplant. In some cases, the patient's liver may begin functioning on its own again after the treatment, thus altogether abrogating the need for liver transplant. The invention is also directed to the use of anti-human immunoglobulin antibodies in the manufacture of a column coupled to said antibodies for the pretreatment of the plasma of a patient prior to ex vivo perfusion of the patient's blood through a pig organ.

EP 0 775 493 A2

**Description**

Each year more than 50.000 patients die of hepatic failure in Europe and the U.S. In the past extracorporeal liver perfusion was tried several times in the treatment of patients with acute or fuliminant liver failure in order to stabilize the clinical condition of those patients. In such systems liver from other species like baboon or pig were applied. Regarding the results of Lie (1992), for concordant liver perfusion using baboon livers a survival rate of about 60% was achieved in humans. Yet, choosing baboon as animal source, there are a lot of disadvantages, like limitations caused by breeding, risk of viral infections , and ethical considerations. Therefore it would be helpful to establish an ex vivo liver perfusion system with organs from discordant animals like pig. To date, however, the main disadvantage in using pig livers is the relatively short period of time (2-6 hours), in which this organ keeps functioning due to hyperacute rejection.

Hereinafter, the term "IA" will refer to immunoapheresis using a column which has a specific ligand coupled thereto, as described below. The term "Ig-THERASORB" will refer to the column which is available from THERASORB Medizinische Systeme GmbH, Unterschleissheim/Munich, Germany. The specific Ig-THERASORB column is also described below.

Methods and compositions for the production of sterile and pyrogen-free protein-coupled columns are provided in WO 95/31727 entitled STERILE AND PYROGEN-FREE COLUMNS COUPLED TO PROTEIN FOR BINDING AND REMOVAL OF SUBSTANCES FROM BLOOD. This application is herein incorporated by reference, with specific reference to the enabling information contained in the following sections: For production of antibodies and virus inactivation, Example 1. For description of pre-columns and working columns, Example 2. For sterile purification of antibodies/protein destined to be coupled to the therapeutic column, Example 3. For preparation of sterile and pyrogen-free column matrix, Example 4. For activation of column matrix material and coupling of protein thereto, Example 5. For finishing of final column product, Example 6.

The Ig-THERASORB column has coupled thereto pooled polyclonal antibodies raised in sheep immunized with pooled human immunoglobulin plus adjuvant. The coupled antibodies bind to human light chains such as lambda and kappa light chains, and thereby recognize and bind to both human IgG and IgM. The coupled antibodies also bind to IgG heavy chain.

Removing xenoreactive antibodies by pre-treatment of the patient's serum with Ig-THERASORB or IgM-THERASORB could prolong the time during which the pig liver would function. Consequently, such an approach would help:

- to bridge the time until an adequate allogeneic liver organ is available for transplantation
- to improve the clinical status of patients, whose liver function is seriously impaired by poisoning or infections such as hepatitis
- in general this approach can also be used if bridging prior to other solid organ transplantations is necessary (i.e. heart)

This method can also be used to increase the functioning time of a transgenic pig liver, for instance a pig liver which expresses human complement inhibitory factors. The method could:

- prevent hyperacute rejection as well as subsequently occuring acute vascular rejection events
- prevent deposition of human antibodies in the pig organ tissue
- result in a less pronounced complement activation
- remove all kinds of antibodies that can cause rejection, irrespective of subtype and specificity

There are several possibilities for the application of Ig-THERASORB or IgM-THERASORB columns, e.g.:

1. Immunoadsorption and ex vivo liver perfusion can be performed separately: first immunoadsortion and then liver perfusion with the possibility to repeat both.

2. Combination of both in an extracorporeal circuit. Blood is separated in a plasma/cell separator (e.g. TPS) in an on-line process. Then the plasma is directed to an apheresis unit, which controls the plasma flow, amount of plasma to be loaded onto one column and column regeneration. After passing one column, the plasma is recombined with the blood cells, perfused through the pig liver and than reintroduced into the patient. This treatment cycle can be repeated as often as it is needed.

Ex vivo liver perfusion using livers from discordant species can be achieved by immunoadsorption alone or in combination with immunosuppression and inhibition of complement activation.

| Example 1 |
| --- |

EXTRACORPOREAL XENOGENEIC PIG LIVER PERFUSION WITH HUMAN BLOOD FOLLOWING ANTIBODY DEPLETION WITH IG-THERASORB

OBJECTIVES

- Extracorporeal xenogeneic pig liver perfusion with human blood, after antibody depletion with Ig-THERASORB columns.

- Extracorporeal xenogeneic pig liver perfusion with human blood, without prior treatment, to establish a control group.

PROCEDURE

Immunoapheresis treatment with 100 ml Ig-THERASORB columns

By means of a primary separation system (e.g. TPS), platelet-free plasma was obtained. The Ig-THERASORB columns were rinsed with 2,000 ml 0.9 % saline per column, in order to remove the storage buffer (PBS/acide). The duration of apheresis treatment depended on IgG concentration. Up to a plasma IgG concentration of ≤ 400 mg/dl the loading volume was set to 260 ml plasma per column cycle. When an IgG concentration of < 400 mg/dl was reached, the loading volume was increased to 520 ml plasma per column cycle. Here the treatment scheme with 100 ml Ig-THERASORB columns and the ADA instrument is described:

1. The loading volume was set to 260 (520) ml plasma per cycle.
2. First cycle

   a. In the first cycle, column I was loaded with 260 (520) ml plasma, column II is inactive.

   b. 120 ml of the loading volume ("Divert Mode") were discarded to waste. This procedure served to remove saline from the column.

   c. After a volume of 120 ml had been discarded, column I was switched to plasma circuit.

   d. After the set plasma volume had passed through column I, the instrument automatically switched to cycle no. 2, and column II was loaded.

3. Second and further cycle(s)

   a. Column II was loaded with plasma, and 120 ml of the loading volume were discarded.

   b. In the meantime, column I was rinsed with 120 ml sterile 0.9 % saline, and the plasma thus removed from the column was reinfused.

   c. After 120 ml had passed through the column, the ADA switched to column II, which was then connected to the plasma circuit while column I was regenerated.

   • d. The regeneration program ran according to the predetermined rinsing volumes set with the ADA number keys, at a flow rate of 100 ml/min

   • e. Rinsing with 10 ml 0.9 % saline.

   - Desorption of the immunoglobulin with 230 ml sterile glycine/HCl buffer, pH 2.8.
   - Neutralization with 300 ml sterile phosphate buffered saline (PBS), pH 7.2.
   - Removal of the PBS by rinsing with 200 ml sterile 0.9 % saline.

- The column was ready for the next adsorption cycle.

f. Loading and rinsing steps in further cycles were performed accordingly.

Before and after the immunoapheresis treatment, plasma samples were collected, and human IgG, IgM and albumin concentrations were determined by nephelometry. The goal was to reach a decrease of the IgG baseline value by ≥ 90 %. The titer of anti-porcine antibodies was determined by an endothelial cell-target ELISA. After treatment, the immunoglobulin depleted human blood further investigated in <u>ex vivo</u> liver perfusion.

⇨ <u>METHOD:</u>

I. Preparation of the perfusate

- Collection of 350-400 ml blood of two healthy donors with identical blood groups and Rhesus subgroups

- Determination of blood group compatibility

- Adjustment of the hematocrit to approx. 30%

- Blood count of the individual donor units and the mixture

- Antibody depletion by means of immunoapheresis

- Blood count following antibody depletion

II. Explantation of the pig's liver (pig's weight: 15-20 kg) under general anesthesia.

III. Preparation of the pig liver, with cannulation of the afferent and efferent vessels and introduction of the organ in the perfusion chamber of the liver perfusion instrument.

IV. Perfusion of the pig liver with antibody-depleted human blood.

⇨ <u>MEASUREMENT VALUES:</u>

- Blood count following blood collection, after mixture of the blood units, hematocrit adjustment and antibody depletion, respectively.

- Blood count at certain times during extracorporeal liver perfusion

- Blood sampling at appropriate times

- At appropriate times, monitoring of the pressure and flow values in the afferent and efferent organ vessels

- At appropriate times, monitoring of the acid/base balance of the perfusion.

- Registration and collection of the bile produced

- Examination and documentation of the macroscopically visible perfusion quality

- Determination of organ weight prior to and after perfusion

- Tissue sampling following perfusion, for light microscopy, electron microscopy and PCR investigations.

⇨ <u>ANALYSIS:</u>

- Determination of the course of liver specific enzyme values, of values of the electrolyte balance and liver synthesis parameters from the blood samples collected at different times, for determination of organ function, perfusion quality and the degree of hyperacute rejection.

- Analysis of the course of oxygen extraction, the acid/base balance, blood count (Hb, Hct, WBC, RBC, PLT) as well as pressure and blood flow values.

- Determination of immunological mediators of hyperacute rejection reaction in the blood samples collected, by ELISA (TNF-alpha, PGF-1 alpha, TXB 2, IL 1, IL 6, IFN-gamma).

- Determination of IgM and IgG titer in the blood samples.

- Immunohistochemical investigation of frozen sections, including deposition of IgM and IgG on vascular endothelium.

- Electrophoresis

- Determination of complement consumption, if applicable.

**RESULT. Extracorporeal xenogeneic pig liver perfusion with human blood following antibody depletion by immunoapheresis**

In 5 test series, cell-free plasma was obtained from human blood (volume: 1 - 1,5 l) by centrifugation (10 min at 3000 rpm). For antibody depletion, 6 immunoapheresis cycles in test series 1-4 were performed, and 7 immunoapheresis cycles in test series 5, using Ig-THERASORB 100 columns/ADA. The loading volume of cycles 1-3 was 260 ml, that of cycles 4-6/7 was 520 ml plasma/cycle (total plasma amount processed: 2.34 l or 2.86 l in test series 5). The following analyses were performed.

- determination of pre-formed anti-pig antibodies, prior to and following apheresis

- nephelometric determination of IgG, IgM and IgA reductions prior to and following apheresis

- nephelometric determination of the complement factors C3, C4 prior to and following apheresis

- determination of TNF-alpha, interleukin-6 and IFN-gamma

- FACS analysis (CD4, CD8, CD3, CD3/HLA-DR, CD19, CD3/CD16 and CD56) prior to and following apheresis, after 30 and 240 minutes of liver perfusion, and after flow of 400 ml effluent, which is pumped through the pig liver prior to perfusion and is then discarded.

1.1 Determination of immunoglobulins, complement factors and pre-formed anti-pig antibodies prior to and following apheresis

**Result**

| Parameter | Immuno-apheresis | Exp. 1. | | Exp. 2 | | Exp. 3 | | Exp. 4 | | Exp. 5 | |
|---|---|---|---|---|---|---|---|---|---|---|---|
| | | conc. mg/ml | red. % | conc. mg/ml | red. % | conc. mg/ml | red. % | conc. mg/ml | red. % | conc. mg/ml | red. % |
| human IgG | before IA | 11.40 | 0 | 10.6 | 0 | 11.7 | 0 | 11.6 | 0 | 9.10 | 0 |
| | after IA | 0.62 | 95 | 0.23 | 98 | 1.12 | 90 | 1.07 | 91 | 0.08 | 99 |
| human IgM | before IA | 1.33 | 0 | 1.27 | 0 | 2.02 | 0 | 2.39 | 0 | 1.68 | 0 |
| | after IA | 0.37 | 72 | 0.32 | 75 | 0.76 | 62 | 0.82 | 66 | <0.31 | <82 |
| human IgA | before IA | 1.37 | 0 | 2.64 | 0 | 2.73 | 0 | 1.71 | 0 | 1.66 | 0 |
| | after IA | 0.21 | 85 | 0.63 | 76 | 0.94 | 66 | 0.50 | 71 | 0.09 | 95 |
| C3 | before IA | 0.78 | 0 | 1.01 | 0 | 0.94 | 0 | 0.87 | 0 | 1.04 | 0 |
| | after IA | 0.38 | 51 | 0.60 | 40 | 0.55 | 41 | 0.54 | 38 | 0.58 | 44 |
| C4 | before IA | 0.14 | 0 | 0.20 | 0 | 0.18 | 0 | 0.14 | 0 | 0.21 | 0 |
| | after IA | 0.08 | 43 | 0.11 | 43 | 0.10 | 44 | 0.08 | 41 | 0.11 | 48 |
| albumin | before IA | 44.5 | 0 | 40.7 | 0 | 44.0 | 0 | 45.6 | 0 | 44.9 | 0 |
| | after IA | 29.5 | 34 | 28.1 | 31 | 27.9 | 37 | 36.5 | 33 | 27.6 | 39 |
| human anti-pig IgG | before IA | | 0 | | 0 | | 0 | | 0 | | 0 |
| | after IA | | 82.1 | | 81.4 | | 70.1 | | 73.6 | | 82.3 |
| human anti-pig IgM | before IA | | 0 | | 0 | | 0 | | 0 | | 0 |
| | after IA | | 56.8 | | 52.7 | | 40.8 | | 24.3 | | 66.4 |

table 1:     **Reduction of immunoglobulins and human IgG/IgM anti-pig antibodies by immunoapheresis with Ig-THERASORB 100**
loading volume of cycle 1-3:     260 ml plasma/cycle
loading volume of cycle 4-6/7:   520 ml plasma/cycle
conc.: concentration
red.: reduction
IA: immunoapheresis

1.2 Determination of immunological mediators of hyperacute rejection reaction

For the detection of immunological mediators of hyperacute and acute rejection reaction, blood samples were collected during pig liver perfusion, and interleukin-6 (IL-6), tumor necrosis factor alpha (TNF-$\alpha$) and interferon-gamma (IFN-$\gamma$) were determined using ELISA tests.

| Sample | Exp. 1 | | | Exp. 2 | | | Exp. 3 | | | Exp. 4. | | | Exp. 5 | | |
|---|---|---|---|---|---|---|---|---|---|---|---|---|---|---|---|
| min | IFN-γ pg/ml | IL-6 pg/ml | TNF-α pg/ml | IFN-γ pg/ml | IL-6 pg/ml | TNF-α pg/ml | IFN-γ pg/ml | IL-6 pg/ml | TNF-α pg/ml | IFN-γ pg/ml | IL-6 pg/ml | TNF-α pg/ml | IFN-γ pg/ml | IL-6 pg/ml | TNF-α pg/ml |
| 0 | 0 | <15 | <25 | 0 | <15 | <25 | 0 | <15 | <25 | 0 | <15 | <25 | 0 | <15 | <25 |
| EF | 0 | <15 | <25 | 0 | <15 | <25 | 0 | <15 | <25 | 0 | <15 | <25 | 0 | <15 | <25 |
| 5 | 0 | <15 | <25 | 18.0 | <15 | <25 | 0 | <15 | <25 | 0 | <15 | <25 | 0 | <15 | <25 |
| 15 | 0 | <15 | 47.3 | 30.0 | <15 | 71.4 | 0 | <15 | 54.9 | 0 | <15 | <25 | 0 | <15 | <25 |
| 30 | 0 | <15 | 112.6 | 21.0 | <15 | 175.9 | 0 | <15 | 161.4 | 18.0 | <15 | 87.5 | 0 | <15 | 70.0 |
| 60 | 0 | <15 | 540.8 | 36.0 | <15 | 645.0 | 141.0 | <15 | 513.7 | 54.0 | <15 | 501.4 | 0 | <15 | 427.1 |
| 90 | 90.0 | <15 | 1483.2 | 36.0 | 66.1 | 1007.7 | 1299.0 | 25.7 | 747.3 | 204.0 | 21.3 | 1599.6 | 0.0 | 15.2 | 1132.5 |
| 120 | 555.0 | 57.4 | 2841.4 | 24.0 | 180.5 | 1305.9 | 1824.0 | 82.7 | 1462.6 | 438.0 | 160.4 | 3661.2 | 63.0 | 75.7 | 2672.6 |
| 180 | 723.0 | 365.8 | 5034.0 | 243.0 | 588.5 | 2563.4 | 1851.0 | 401.8 | 2694.0 | 615.0 | 622.4 | 6991.6 | 285.0 | 412.5 | 5614.8 |
| 240 | 723.0 | 569.7 | 6255.1 | 351.0 | 953.9 | 2837.0 | 1545.0 | 827.2 | 3140.3 | 513.0 | 805.4 | 7408.1 | 213.0 | 619.5 | 6235.8 |

table 2: **Determination of immunological mediators of hyperacute rejection reaction, prior to, during and after extracorporeal xenogeneic pig liver perfusion with antibody-depleted human blood**

EF: Effluate
IL-6: 15 pg/ml lowest default measuring value
TNF-α: 25 pg/ml lowest default measuring value

Result

FACS Analyses

The objective of FACS analyses is to observe changes in the distribution (percentage) of lymphocyte populations during liver perfusion. For FACS analysis, fresh EDTA blood is incubated with FITC- or PE-labeled antibodies. Then the erythrocytes are subjected to lysis, and the leukocytes are purified by repeated washing steps with PBS. The so treated leukocytes are analyzed with the Becton Dickinson's FACScan, using SimulSET Software. The results of tests 2,3 and 4 are summarized in table 4. As is already known from experiments with ITP patients, immunoapheresis alone does not cause any changes. Further investigations are required in order to be able to make statements about changes during the course of perfusion. The FACS results should be assessed with regard to the differential blood count. To achieve a better assessment of results, the following table 3 states the normal ranges of the parameters determined:

| Cell population | Normal distribution |
| --- | --- |
| total T cells | 59.4-84.6 |
| activated T cells | no information |
| Helper/Inducer T cells | 29.0-59.0 |
| Suppressor/Cytotoxic T cells | 19.0-48.0 |
| Ratio Helper/Suppressor | 0.9-3.6 |
| total B cells | 6.4-22.6 |
| NK cells | 5.6-30.9 |

table 3: **Normal values of the cell parameters determined**
The listed normal ranges have been taken from the manufacturer´s documentation. The data refer to the distribution of lymphocyte population in the normal Caucasian population.

| Sample | T-Cells | | | | | B-Cells | NK-Cells |
|---|---|---|---|---|---|---|---|
| | total CD3+ | activated. C3/HLA-DR | Helper/Ind. CD4 | Supp./Cyt CD8 | Ratio CD4/CD8 | CD 19 total | CD3/CD16 +CD56 |
| | % | % | % | % | % | % | % |
| **2. Experiment** | | | | | | | |
| Donor 1 | 69 | 6 | 40 | >56 | 0.71 | 9 | 22 |
| Donor 2 | 72 | 8 | 51 | 30 | 1.7 | 15 | 12 |
| Donor pool | 71 | 5 | 40 | 40 | 1.0 | 11 | 15 |
| after IA | 72 | 8 | 42 | 42 | 1.0 | 13 | 14 |
| 400 ml EF | <19 | 2 | <14 | <11 | 1.3 | <3 | <4 |
| after 30 min | <9 | 1 | <8 | <2 | 4.0 | <1 | <1 |
| End of perfusion | <11 | 0 | <7 | <4 | 2.0 | <1 | <1 |
| **3. Experiment** | | | | | | | |
| Donor 1 | 69 | 3 | 50 | 32 | 1.6 | 10 | 19 |
| Donor 2 | 71 | 6 | >60 | 42 | 1.4 | 8 | 22 |
| Donor 3 | 66 | 12 | 57 | 40 | 1.4 | 11 | 23 |
| Donor pool | 69 | 12 | >62 | 36 | 1.7 | 9 | 23 |
| after IA | 71 | 12 | >62 | 40 | 1.6 | 10 | 21 |
| 400 ml EF | 73 | 7 | 56 | 30 | 1.5 | 10 | 25 |
| after 30 min | <8 | 1 | <8 | <4 | 2.0 | <1 | <1 |
| End of perfusion | <45 | 5 | <26 | 24 | 1.1 | <6 | 9 |
| **4. Experiment** | | | | | | | |
| Donor pool | 73 | 10 | 47 | 38 | 1.2 | 11 | 16 |
| after IA | 69 | 8 | 44 | 42 | 1.0 | 10 | 22 |
| 400 ml EF | 74 | 8 | 51 | 41 | 1.2 | 14 | 28 |
| after 30 min | <4 | 0 | <3 | <1 | 3.0 | <2 | <1 |
| End of perfusion | <3 | 6 | <9 | <6 | 1.5 | <5 | <2 |

**table 4:** Analysis of the distribution (percentage) of lymphocyte populations

**IA: immunoapheresis** **Ind.: Inducer**

**EF: Effluent** **Supp.: Suppressor**

**Cyt.: Cytotoxic** **NK: Natural Killer**

---

**Example 2. Extracorporeal xenogeneic pig liver perfusion with human blood following treatment with uncoupled sepharose (control tests)**

---

In order to establish a control group, 5 test series were performed. Analogous to test series 1, cell-free plasma was obtained from human blood (blood volume 1 - 1.5 l) by centrifugation (10 min at 3000 rpm). The plasma was then processed using non-antibody-coupled sepharose material. Afterwards, it was mixed again with the blood cells and extracorporeal xenogeneic liver perfusion was performed.

2.1 Determination of immunoglobulins, complement factors and pre-formed anti-pig antibodies (control group)

**Result**

| Parameter | plasma processing | Exp. 1 | | Exp. 2 | | Exp. 3 | | Exp. 4 | | Exp. 5 | |
|---|---|---|---|---|---|---|---|---|---|---|---|
| | | conc. mg/ml | red. % | conc. mg/ml | red. % | conc. mg/ml | red. % | conc. mg/ml | red. % | conc. mg/ml | red. % |
| human IgG | before | 10.50 | 0 | 9.24 | 0 | 12.05 | 0 | 9.06 | 0 | 10.90 | 0 |
| | after | 7.05 | 32.9 | 6.84 | 26.0 | 8.58 | 28.8 | 6.16 | 32.0 | 7.97 | 26.9 |
| human IgM | before | 1.23 | 0 | 1.88 | 0 | 1.93 | 0 | 1.86 | 0 | 1.90 | 0 |
| | after | 0.75 | 39.0 | 1.21 | 35.6 | 1.16 | 39.9 | 1.00 | 46.2 | 1.26 | 33.7 |
| human IgA | before | 1.68 | 0 | 2.19 | 0 | 2.29 | 0 | 1.60 | 0 | 2.47 | 0 |
| | after | 1.13 | 32.7 | 1.62 | 26.0 | 1.60 | 30.1 | 1.06 | 33.8 | 1.77 | 28.3 |
| C3 | before | 0.92 | 0 | 0.91 | 0 | 1.05 | 0 | 0.92 | 0 | 0.95 | 0 |
| | after | 0.66 | 28.3 | 0.65 | 28.6 | 0.70 | 33.3 | 0.63 | 31.5 | 0.68 | 28.4 |
| C4 | before | 0.16 | 0 | 0.18 | 0 | 0.16 | 0 | 0.16 | 0 | 0.18 | 0 |
| | after | 0.11 | 31.3 | 0.12 | 33.3 | 0.10 | 37.5 | 0.10 | 37.5 | 0.12 | 33.3 |
| albumins | before | 49.8 | 0 | 39.85 | 0 | 46.85 | 0 | 48.00 | 0 | 40.55 | 0 |
| | after | 32.95 | 33.8 | 31.10 | 22.0 | 33.25 | 29.0 | 33.25 | 30.7 | 30.00 | 26.0 |
| human anti-pig IgG | before | | 0 | | 0 | | 0 | | 0 | | 0 |
| | after | | 11.1 | | 8.6 | | 15.7 | | 27.5 | | 21.2 |
| human anti-pig IgM | before | | 0 | | 0 | | 0 | | 0 | | 0 |
| | after | | 13.3 | | 15.7 | | 21.4 | | 16.9 | | 14.9 |

table 5: **Determination of immunoglobulins (IgG,-A,-M), complement factors (C3, C4) and human IgG/IgM anti-pig antibodies in plasma, prior to and after treatment with uncoupled sepharose (100 ml columns).**
loading volume of cycle 1-3: 260 ml plasma/cycle
loading volume of cycle 4-6: 520 ml plasma/cycle
conc.: concentration
red.: reduction
IA: immunoapheresis

Table 6 and 7 includes the measuring values of five experiments each with the investigational and control groups. The results suggest that immunoapheresis with Ig-THERASORB columns are a safe and effective technology for removing anti-porcine IgG and IgM antibodies from human plasma. The human IgG level decreased by 95%, the IgM level by 71% and IgA level by 79%. The xenoreactive antibodies were reduced to 78% for human anti-pig IgG and 48% for human anti-pig IgM. The reduction of the immunoglobulins and xenoreactive antibodies of the control experiments with uncoupled Sepharose columns presented in table 7 results from the procedure-dependent plasma dilution or plasma loss. By improving this experimental setting for clinical use the observed dilutional effects could be further reduced.

|  | immunoglobulin | | | xenoreactive antibody | |
| --- | --- | --- | --- | --- | --- |
|  | IgG | IgM | IgA | IgG | IgM |
| reduction | 95% | 71% | 79% | 78% | 48% |

table 6: **Determination of immunoglobulins (IgG,-A,-M), and human IgG/IgM anti-pig antibodies in plasma, prior to and after treatment with Ig-THERASORB columns**
Investigational group n = 5

|  | immunglobulin | | | xenoreactive antibody | |
| --- | --- | --- | --- | --- | --- |
|  | IgG | IgM | IgA | IgG | IgM |
| reduction | 29% | 39% | 30% | 17% | 16% |

table 7: **Determination of immunoglobulins (IgG,-A,-M), and human IgG/IgM anti-pig antibodies in plasma, prior to and after treatment with uncoupled sepharose (100 ml columns)**
Control group n = 5

2.2 Determination of immunological mediators of the hyperacute rejection reaction

For the detection of immunological mediators of hyperacute and acute rejection reaction, blood samples were collected during pig liver perfusion, and interleukin-6 (IL-6), tumor necrosis factor alpha (TNF-$\alpha$) and interferon gamma were determined using ELISA tests.

EP 0 775 493 A2

| Sample | Exp. 1 | | | Exp. 2. | | | Exp. 3. | | | Exp. 4. | | | Exp. 5. | | |
|---|---|---|---|---|---|---|---|---|---|---|---|---|---|---|---|
| min | IFN-γ U/ml | IL-6 pg/ml | TNF-α pg/ml | IFN-γ U/ml | IL-6 pg/ml | TNF-α pg/ml | IFN-γ U/ml | IL-6 pg/ml | TNF-α pg/ml | IFN-γ U/ml | IL-6 pg/ml | TNF-α pg/ml | IFN-γ U/ml | IL-6 pg/ml | TNF-α pg/ml |
| 0 | 0 | <15 | <25 | 0 | <15 | <25 | 0 | <15 | <25 | 0 | <15 | <25 | 0 | <15 | <25 |
| EF | 0 | <15 | <25 | 0 | <15 | <25 | 0 | <15 | <25 | 0 | <15 | <25 | 0 | <15 | <25 |
| 5 | 0 | <15 | <25 | 0 | <15 | <25 | 0 | <15 | <25 | 0 | <15 | <25 | 0 | <15 | <25 |
| 15 | 0 | <15 | <25 | 0 | <15 | <25 | 0 | <15 | <25 | 0 | <15 | <25 | 0 | <15 | <25 |
| 30 | 0 | <15 | 61.6 | 0 | <15 | 60.0 | 0 | <15 | 70.6 | 0.06 | <15 | 127.7 | 0 | <15 | 51.8 |
| 60 | 0 | <15 | 504.8 | 0 | <15 | 319.6 | 0 | <15 | 450.9 | 0 | <15 | 727.9 | 0 | <15 | 643.8 |
| 90 | 0 | 45.8 | 1168.4 | 0 | 47.8 | 773.9 | 0 | 43.0 | 1190.3 | 0 | 42.8 | 2026.7 | 0 | 48.2 | 1661.4 |
| 120 | 150.0 | 120.2 | 1706.1 | 111.0 | 132.1 | 1206.0 | 168.0 | 110.2 | 1859.6 | 123.0 | 121.2 | 4154.1 | 159.0 | 188.8 | 3571.3 |
| 180 | 579.0 | 391.8 | 2627.4 | 159.0 | 311.7 | 1800.0 | 186.0 | 468.9 | 3385.8 | 81.0 | 380.1 | 5922.0 | 111.0 | 861.7 | 5796.9 |
| 240 | 177 | 544.0 | 2834.3 | 0 | 304.9 | 2000.0 | 0 | 640.6 | 3624.0 | 54.0 | 829.9 | 5974.6 | 129.0 | 1034.9 | 6148.7 |

table 8:     **Determination of immunological mediators of the hyperacute rejection reaction, prior to, during and after extracorporeal xenogeneic pig liver perfusion with human blood, <u>without</u> antibody depletion (control experiments)**
EF:          Effluent
IL-6:        15 pg/ml lowest default measuring value
TNF-α:       25 pg/ml lowest default measuring value

<u>Result</u>

13

2.3 FACS Analyses

The percentage distribution of various lymphocyte populations in the plasma samples prior to and after plasma processing, as well as after 30 and 240 minutes (i.e. at the end) of liver perfusion, was determined with FACS. So far, the plasma samples have only been coupled with two fluorescent dyes (PE, FITC) coupled to monoclonal antibodies. The cell population to be investigated, i.e. the lymphocytes, were separated from other cells according to size (forward scatter) and granularity (side scatter) using an analysis window (gate). But with this method cellular contaminants (debris) of similar size or granularity were included as well. Debris was so significant in plasma samples of the first test series with Ig-THERASORB - after 30 minutes perfusion in the gate - that a statistical evaluation of the few lymphocytes was impossible with the SimulSET software. This problem was avoided in the analysis of the control sample by using a third fluorescent dye (PerCP). The antibody coupled to this dye binds specifically to the leukocyte surface antigen CD45. By PerCP marking, the leukocytes could be separated from debris in the analysis gate and were analyzed with the program LYSYS II.

Prior to marking the plasma samples, the leukocytes contained in them were counted using a hemacytometer. For this purpose, an aliquot of the sample to be tested was diluted 1:2 with a dye solution (crystal purple), thus triggering an erythrocyte lysis. 10 µl of the solution were pipetted into the hemacytometer and were investigated using a microscope with 100 x total magnification. The leukocyte concentration was calculated as follows: mean of the cells/large quadrant X dilution of the sample x $10^4$ = cells x $10^6$/ml .

The following table shows the result of the plasma sample count of test 1, April 24, 1995. The samples of the other experiments (2-5) were not counted.

| Sample | Q1 | Q2 | Q3 | Q4 | Mean | $10^6$ cells/ml |
|---|---|---|---|---|---|---|
| prior to processing | 174 | 159 | 168 | 167 | 176.0 | 3.34 |
| after processing | 168 | 165 | 117* | 167 | 166.7 | 3.33 |
| after 30 min perf. | 37 | 32 | 37 | 40 | 36.5 | 0.73 |

table 9: **Leukocyte number in plasma samples of control experiment 1 (April 24, 1995)**
* was not considered in the mean
Q = Large quadrant of the hemacytometer
perf.= Perfusion

As shown in the table, the leukocyte numbers prior to and after plasma processing are practically identical. However, after 30 min of liver perfusion, only approx. 22 % of the leukocytes can be detected microscopically.

In order to be able to mark a sufficient amount of leukocytes for FACS analysis, also in those samples taken during or after perfusion, (according to B&D 3.5 x $10^5$ - 9.8 x $10^5$ cells are required per sample), 500 µl aliquots of all plasma samples are centrifuged (Baxter Desktop Centrifuge, 1.5 min, high), the pellet is resuspended in 80 µl PBS and, as usual, 20 µl Simultest solution (PE- or FITC-labeled antibody) are added. In addition, the sample is mixed with 20 µl PerCP-labeled anti-CD45 antibody (exception: Isotype control sample). The further procedure was performed according to the usual scheme. The final pellet was added to 300 µl paraformaldehyde.

The following table summarizes the results of the FACS analysis of the samples of tests 2 and 3. The samples of tests 1 and 4 could not be analyzed for technical and practical reasons.

| Sample | T-Cells | | | | | B-Cells | NK-Cells |
|---|---|---|---|---|---|---|---|
| | total CD3+ | activated C3/HLA-DR | Helper/Ind. CD4 | Supp./Cyt CD8 | Ratio CD4/CD8 | CD 19 total | CD3/CD16+ CD56 |
| | % | % | % | % | % | % | % |
| **Exp. 2** | | | | | | | |
| **(25.04.95)** | | | | | | | |
| before plasma processing | 38.7 | 1.4 | 30.8 | 20.4 | 1.5 | 5.4 | 36.1 |
| after plasma processing | 30.3 | 1.0 | 23.1 | 15.5 | 1.5 | 4.6 | 41.5 |
| mean value before/after | 34.5 | 1.2 | 27.0 | 18.0 | 1.5 | 5.0 | 38.8 |
| after 30 min | 92.1 | 3.8 | 75.6 | 19.1 | 4.0 | 0.8 | 4.6 |
| end of perfusion | 78.4 | 15.8 | 73.6 | 34.3 | 2.2 | 1.3 | 2.1 |
| **Exp. 3** | | | | | | | |
| **(26.04.95)** | | | | | | | |
| before plasma processing | 47.1 | 7.5 ? | 37.6 | 37.3 | 1.0 | 3.4 | 22.9 |
| after plasma processing | 38.1 | 2.4 | 34.4 | 28.4 | 1.2 | 3.1 | 27.6 |
| mean value before/after | 42.6 | 2.4 | 36.0 | 32.9 | 1.1 | 3.3 | 25.3 |
| after 30 min | 88.2 | 8.0 | 71.6 | 22.5 | 3.2 | 1.8 | 5.2 |
| end of perfusion | n.e. | n.e. | n.e. | n.e. | n.e. | n.e. | n.e. |

table 10:   **Distribution (%) of lymphocyte populations**
Analysis using three color immunofluorescence in FACSsort
Ind.:       Inducer
Supp.:      Suppressor
Cyt.:       Cytotoxic
NK:         Natural Killer
n.e.:       Sample "end of perfusion" of experiment 3
was not evaluated since no lymphocyte
population could be detected.

As shown by the table, the distribution (percentage) of the lymphocyte populations during plasma processing remained practically unchanged. However, after 30 min of liver perfusion the total number of T-cells had already multiplied by 2.7 or 2.1, respectively. At the same time, activated T-cells had multiplied by 3.3 times. The number of CD4-positive cells multiplied by 2.4 times on average, that of CD8-positive cells only 1.3 times. Thus the CD4/CD8 ratio increased from 1.3 to 2.8.

The percentage of B lymphocytes decreased from 5.0 or 3.3 % to 0.8 or 1.8 %, respectively. The percentage of natural killer cells decreased by factor 5-8.

## Example 3  Ex vivo liver perfusion

### Method

- Explantation of the liver of a pig (pig's weight: 20 - 25 kg) under general anesthesia, in connection with multiorgan explantation (liver, heart and kidneys) following a rinsing step with 4°C University of Wisconsin Solution

- Preparation of the pig liver, with cannulation of the afferent and efferent vessels

- Introduction of the liver into the perfusion chamber and subsequent 4 hour perfusion with human blood

- During the whole perfusion period: monitoring and documentation of pressure, flow and resistance values in the organ vessels

- During the whole perfusion period: monitoring of oxygen saturation and acid/base balance

- Collection of blood samples in different special containers at 10 predetermined points in time

- Collection and documentation of the bile produced

- Monitoring and documentation of the macroscopically visible perfusion quality

- Determination of organ weight prior to and after perfusion

- Tissue sampling following perfusion, for light and electron microscopy and immunohistochemical investigations

### Evaluation

- Procedure analysis of oxygen extraction, acid/base balance, flow, pressure and resistance values.

- Analysis of changes in the blood count and differential blood count.

- Determination of hemagglutinin titer and electrophoresis investigations of the samples

- Procedure analysis of liver specific enzyme values, synthesis parameters and electrolyte balance, for the assessment of tissue damage, organ function, perfusion quality and the degree of hyperacute rejection reaction:

    Parameters for cellular damage and functional impairments (ALAT, ASAT, GLDH, CPK, LDH, AP, gGT, bilirubin, potassium)

    Parameters for synthesis performance (glucose, albumin, urea, bile production)

    Parameters for metabolic performance(BST clearance and others)

- Measurement of weight increase for quantification of interstitial edema formation during rejection reaction

    Light and electron microscopy investigation of tissue morphology following perfusion, for localization and assessment of tissue damage

- Immunohistochemical investigation of frozen sections

Since the evaluation process of some parameters has not yet been completed, the above is only a part of the analysis.

Results

Five experiments each were assessed for the control and investigational groups.

The transaminases GOT and GPT as well as the liver specific enzyme GLDH, which are the classical parameters of human medicine for the assessment of hepatocellular integrity, are also the parameters used for our model. Due to their relatively characteristic distribution in different cell compartments, their concentration in serum allows for certain conclusions concerning the degree of cellular damage. The investigations made so far suggest a significant difference between control and investigational. The determination of creatine phosphate kinase and potassium, which are mainly located in the intracellular space, indicated minor cellular damage to the pig liver in the investigational group (i.e. following immunoapheresis with Ig-THERASORB).

Bile production is the most significant parameter for clinical assessment of liver function. It was much higher in the investigational group than in the control group, indicating that Ig-THERASORB pre-treatment of human plasma spared the pig liver function to a great extent.

The same finding applied to another synthesis parameter: urea production (endpoint of amino acid metabolism).

The bromosulphtalein clearance test, a dye elimination test for the evaluation of the liver's detoxification capacity, indicated a higher residual detoxification capacity of the organs in the investigational group. This is highly significant for future clinical use.

Edema formation, indicated by higher weight increase in the investigational pig livers, was an indirect sign of endothelial damage. It is understood that it may be necessary to exchange pig livers when each one shows signs of damage due to immune rejection. However, these data indicate that pre-treatment of patient plasma with Ig-THERASORB can preserve the function of an individual pig liver for up to 4 hours, and perhaps longer. The experiments were arbitrarily stopped at 4 hours. At this time point, three indicators of liver function suggested that the livers could have kept on functioning for much longer.

---

## Example 4

## Immunoapheresis in the clinical setting.

---

Methods and compositions for the production of sterile and pyrogen-free protein-coupled columns are provided in the co-owned U.S. patent application serial number 08/242,208 entitled STERILE AND PYROGEN-FREE COLUMNS COUPLED TO PROTEIN FOR BINDING AND REMOVAL OF SUBSTANCES FROM BLOOD, filed May 13, 1994. This application is herein incorporated by reference, with specific reference to the enabling information contained in the pages as follows:

For production of antibodies and virus inactivation, Example 1, pages 15-19.

For description of pre-columns and working columns, Example 2, pages 19-20.

For sterile purification of antibodies/protein destined to be coupled to the therapeutic column, Example 3, pages 20-25.

For preparation of sterile and pyrogen-free column matrix, Example 4, pages 26-27.

For activation of column matrix material and coupling of protein thereto, Example 5, pages 27-30.

For finishing of final column product, Example 6, page 30.

The following describes the method which can be used to pretreat the blood of the patient for extracorporeal organ perfusion. An instrument can be designed to pump the blood, oxygen, and other solutions through the pig liver.

Anti-human immunoglobulin coupled columns were used for the removal of immunoglobulin from the blood of human patients suffering from idiopathic thrombocytopenic purpura (ITP), systemic lupus erythematosus (SLE), vasculitis, and sensitizatiion to HLA. These procedures were part of controlled clinical trials carried out in Europe for the treatment of autoimmune patients whose conditions were refractory to conventional treatments, and patients in need of kidney transplant who had cytotoxic anti-HLA antibodies in their blood.

Briefly, the tubing system of the primary separation system was first filled with sterile 0.9% NaCl. Two anti-human Ig columns (Ig-THERASORB, Baxter, Immunotherapy Division, Europe) were connected with the primary separation system. All tubing connections were made under aseptic conditions.

To remove the preservative solution from the columns, each column was rinsed before its first use with 5 liters sterile 0.9% NaCl solution, at a flow rate of 90-100 ml/min. For each subsequent use, it was sufficient to rinse each column with 2 liters of the sterile solution, at the same flow rate.

Before start of the procedure, the entire system was tested for absence of air bubbles and leaks, correct connections of the solutions, including the anticoagulants, correct installation of the programming of the device, functionality of the automatic clamps, and the safety system.

The appropriate canulae were connected to the left and right cubital veins of the patient. Blood samples were taken. The connection to the blood cell separator was put in place. Anticoagulation was accomplished with either heparin or citrate (ACD-A or ACD-B) or a combination of both. When citrate was the anti-coagulant, during the first half of the procedure, the citrate was used at a dilution of 1:22 to 1:18. In the second therapy phase, the dilution utilized was 1:12 to 1:8. Hypocalcemia is known to be a common side-effect associated with the use of a different type of column (Protein-A coupled column) because the Protein A column protocol requires returning citrate buffer to the patient. In the present protocol for use of Ig-THERASORB, citrate buffer is not returned to the patient. However, during use of the Ig-Thersorb column, symptoms of hypocalcemia were monitored (paraesthesia in fingers or lips) so that the administration of anti-coagulant citrate could be diminished accordingly. There were no reports that reduction of anticoagulant was necessary. Calcium tablets were kept at hand to be given in case of frank hypocalcemia, but there were no reports of any necessity to administer calcium tablets.

After the venous puncture and the connection of the tubing system to the patient, the blood cell separator was filled with the patient's blood. The blood flow rate was kept between 50-90 ml/min. When a column with a volume of 100 ml was used, the liquid level was maintained at about 0.8 cm over the Sepharose_ in the column. After the stabilization of the separation process, the cell-free plasma was directed through the tubing system over the first column. It was important to keep the flow rate even and to monitor the plasma level over the Sepharose_ in the column. A higher plasma level was undesirable, because it would have led to a higher volume burden for the patient, and plasma loss due to plasma retention in the column.

Using a plasma flow rate of up to 40 ml/min, the column was loaded with as much plasma as possible during 15 minutes. Thereafter, the plasma flow was switched to the second column, which was likewise filled with as much plasma as possible in 15 minutes. The volume of processed plasma per cycle was 260 ml at the beginning and then 520 ml (see table 11).

During the time of filling of the second column, the plasma in the first column was flushed out using sterile 0.9% NaCl at the plasma flow rate. One column volume of plasma was returned to the patient together with the blood cells which had been removed.

Also during filling of the second column, the first column was regenerated as follows: (1) A further rinse with 50 ml 0.9 % NaCl at a flow rate of 100 ml/min; (2) Desorption of the bound immunoglobulin with one column volume of sterile 0.2 M glycine/HCl buffer, pH 2.8. The controller of the device prevented contact between this solution and the patient. The desorbed immunoglobulin was discarded. (3) Neutralization with three column volumes of sterile PBS, pH 7.4. Testing of the neutralization using pH indicator paper. (4) Rinsing out of the PBS with at least one column volume of sterile 0.9% Nacl. The column was then ready for the next round of adsorption. Then, the filling of the columns was again automatically switched. This procedure was repeated as many times as necessary to process the desired volume of plasma. The number of cycles used was chosen by the attending physician according to our kinetic data (table 11). So far, within the inventors' clinical experience, it has been possible to process up to 3.5 times the extracorporeal volume of a given patient during one column procedure. Moreover, the number of cycles used was not limited by the binding capacity of the columns, but rather by the needs of the individual patient.

Blood samples were taken for analysis of the success of the procedure. Assays for immunoglobulin subclasses were performed, and panel reactivity (PR) tests were done for those patients who had anti-HLA antibodies.

After each procedure, the columns assigned to each patient were cleaned and stored under aseptic conditions at 2-8°C until the next use for the same patient. Results: Preliminary results showed that the IgG concentration in the subjects' blood was reduced by at least 70% to 99% compared to starting concentrations. IgA and IgM levels were reduced by 70% to 90%. Thus, pre-formed naturally occurring antibodies as well as all kinds of alloantibodies which contribute to graft rejection were adsorbed by the column.

There was no morbidity or mortality associated with the use of the column procedure. Plasma loss was typically low (4-8%), and no plasma replacement was required.

number of cycles ⎯ $\begin{bmatrix}12 \times 260\\2 \times 520\end{bmatrix}$ ⎯ processed plasma [ml] per cycle

equations (1): $f(c) = 0.7 + 2.79 \times C + \frac{2}{30000}(2.34)^{C}$

(2): $V_{proc} = f(15 - C_{after}) - f(15 - C_{before})$

desired
IgG [mg/dl]
after apheresis

| | 1500 | 1400 | 1300 | 1200 | 1100 | 1000 | 900 | 800 | 700 | 600 | 500 | 400 |
|---|---|---|---|---|---|---|---|---|---|---|---|---|
| 1400 | 3 x 260 | | | | | | | | | | | |
| 1300 | 6 x 260 | 3 x 260 | | | | | | | | | | |
| 1200 | 9 x 260 | 6 x 260 | 3 x 260 | | | | | | | | | |
| 1100 | 12 x 260 | 9 x 260 | 6 x 260 | 3 x 260 | | | | | | | | |
| 1000 | 15 x 260 | 12 x 260 | 9 x 260 | 6 x 260 | 3 x 260 | | | | | | | |
| 900 | 18 x 260 | 15 x 260 | 12 x 260 | 9 x 260 | 6 x 260 | 3 x 260 | | | | | | |
| 800 | 20 x 260 | 18 x 260 | 15 x 260 | 12 x 260 | 9 x 260 | 6 x 260 | 3 x 260 | | | | | |
| 700 | 23 x 260 | 20 x 260 | 18 x 260 | 15 x 260 | 12 x 260 | 9 x 260 | 6 x 260 | 3 x 260 | | | | |
| 600 | 26 x 260 | 23 x 260 | 20 x 260 | 18 x 260 | 15 x 260 | 12 x 260 | 9 x 260 | 6 x 260 | 3 x 260 | | | |
| 500 | 29 x 260 | 26 x 260 | 23 x 260 | 20 x 260 | 18 x 260 | 15 x 260 | 12 x 260 | 9 x 260 | 6 x 260 | 3 x 260 | | |
| 400 | 32 x 260 | 29 x 260 | 26 x 260 | 23 x 260 | 20 x 260 | 18 x 260 | 15 x 260 | 12 x 260 | 9 x 260 | 6 x 260 | 3 x 260 | |
| 300 | 32 x 260 / 2 x 520 | 29 x 260 / 2 x 520 | 26 x 260 / 2 x 520 | 23 x 260 / 2 x 520 | 20 x 260 / 2 x 520 | 18 x 260 / 2 x 520 | 15 x 260 / 2 x 520 | 12 x 260 / 2 x 520 | 9 x 260 / 2 x 520 | 6 x 260 / 2 x 520 | 3 x 260 / 2 x 520 | 2 x 520 |
| 200 | 32 x 260 / 4 x 520 | 29 x 260 / 4 x 520 | 26 x 260 / 4 x 520 | 23 x 260 / 4 x 520 | 20 x 260 / 4 x 520 | 18 x 260 / 4 x 520 | 15 x 260 / 4 x 520 | 12 x 260 / 4 x 520 | 9 x 260 / 4 x 520 | 6 x 260 / 4 x 520 | 3 x 260 / 4 x 520 | 4 x 520 |

TABLE 11: Immunoapheresis procedure with Ig-Therasorb columns
Example for patients with 3500 ml extracorporeal plasma volume
Equations 1 and 2: C: concentration of IgG [mg/ml], $V_{proc}$: processed plasma [ml]

**Claims**

1. A method of perfusing blood comprising:

    (a) providing a sterile and pyrogen-free column coupled to anti-human immunoglobulin antibodies,
    (b) separating the plasma from the blood cells of the patient,
    (c) passing the plasma of the patient over the column under conditions which effect the binding of said human immunoglobulin antibodies to immunoglobulin in the patient's plasma, thereby removing a significant portion of the immunoglobulin from said patient's plasma,
    (d) recombining the patient's immunoglobulin-depleted plasma with the patient's blood cells to form immnunoglobulin-delepleted blood, and
    (e) perfusing the patient's immunoglobulin-depleted blood through a pig organ.

2. The method of Claim 1, wherein said pig organ is a liver or a heart.

3. The use of anti-human immunoglobulin antibodies in the manufacture of a column coupled to said antibodies for the pre-treatment of the plasma of a patient prior to *ex vivo* perfusion of the patient's blood through a pig organ.

4. A method of removing xenoreactive antibodies from serum comprising passing the serum over a sterile and pyrogen-free column coupled to anti-human immunoglobulin antibodies.

5. A column for removing xenoreactive antibodies from plasma, the column being sterile and pyrogen free and being coupled to anti-human immunoglobulin antibodies.

6. The use of anti-human immunoglobulin antibodies in the manufacture of a column coupled to said antibodies for treating plasma of a patient for the purpose of improving the clinical status of a patient with liver disease.